**Europäisches Patentamt**

**European Patent Office**

**Office européen des brevets**

(11) Veröffentlichungsnummer: **0 012 959**
**B1**

(19)

(12) EUROPÄISCHE PATENTSCHRIFT

(45) Veröffentlichungstag der Patentschrift:
05.05.82

(21) Anmeldenummer: 79105190.7

(22) Anmeldetag: 14.12.79

(51) Int. Cl.³: **A 61 F 1/00X** // A61K35/32, A61K37/12

(54) Verfahren zur Herstellung von Knochenersatzmaterial mit verbesserter biologischer Stabilität auf der Basis von Kollagen und das erhaltene Knochenersatzmaterial.

(30) Priorität: 16.12.78 DE 2854490

(43) Veröffentlichungstag der Anmeldung:
09.07.80 Patentblatt 80/14

(45) Bekanntmachung des Hinweises auf die Patenterteilung:
05.05.82 Patentblatt 82/18

(84) Benannte Vertragsstaaten:
AT BE CH DE FR GB IT LU NL SE

(56) Entgegenhaltungen:
DE-C-961 854
GB-A-1 068 587

CHEMICAL ABSTRACTS, Band 84, Nr. 16,
Seite 409, Spalte 2, Nr. 111690d, 19. April 1976
Columbus, Ohio, USA

(73) Patentinhaber: Intermedicat GmbH, Gerliswilstrasse 45,
CH-6020 Emmenbrücke (CH)

(72) Erfinder: Werner, Heinz-Helmut Dr. med., Am Steig 2,
D-3508 Melsungen (DE)

(74) Vertreter: von Kreisler, Alek, Dipl.-Chem. et al,
Deichmannhaus am Hauptbahnhof, D-5000 Köln 1 (DE)

## Verfahren zur Herstellung von Knochenersatzmaterial mit verbesserter biologischer Stabilität auf der Basis von Kollagen und das erhaltene Knochenersatzmaterial

Die Erfindung betrifft ein Verfahren zur Herstellung von Knochenersatzmaterial mit verbesserter biologischer Stabilität auf der Basis von Kollagen und ein nach diesem Verfahren hergestelltes Knochenmaterial.

In der Chirurgie besteht immer wieder die Notwendigkeit, Knochendefekte mit einem Füllmaterial zu versehen. Dies ist vor allem notwendig nach Unfallverletzungen, die zu Knochenbrüchen mit Knochensubstanzverlust oder Knocheneinstauchung geführt haben. Es gibt jedoch zahlreiche weitere Anwendungen, wie z.B. Defektfüllung nach Entfernung von Knochentumoren oder Ausräumung von Knochenzysten. Weiterhin wird ein Knochenfüllmaterial in den Fällen benötigt, in denen der Chirurg im Rahmen von Korrekturmassnahmen am Knochen einen bereits vorhandenen oder von ihm selbst geschaffenen Hohlraum auffüllen muss.

Nach dem derzeitigen Stand der chirurgischen Technik ist das bestgeeignete Material autologe Spongiosa oder ein autologer kortiko spongiöser Span [Literatur: Unfallheilkunde, 81, 565 - 567 (1978)]. In diesem Fall ist der Empfänger zugleich auch der Spender des Knochens.

Die Übertragung von menschlichen Knochen von einer Person auf eine andere, die homologe Transplantation, ist ebenfalls gebräuchlich. Hierzu sind stellenweise Knochenbanken eingerichtet, die geeignetes Material bereithalten. Des weiteren ist die Übertragung von tierischen Knochen, der einem geeigneten Aufbereitungsverfahren unterworfen wurde, wie z.B. Einlagerung in Cialitlösung oder nach dem in der DE-C-961 654 angegebenen Verfahren, bekannt und gebräuchlich.

Die besten chirurgischen Ergebnisse am Einpflanzungsort werden bis jetzt ohne jeden Zweifel dann erzielt, wenn vom gleichen Patienten, der dieses Material erhalten soll, auch der Knochen entnommen wird (autologe Transplantation). Hierbei ergeben sich zwei entscheidende Nachteile:

1. Das Knochenersatzmaterial muss hier durch eine zusätzliche Operation gewonnen werden. Der zusätzliche operative Eingriff führt zu einer Verlängerung der Gesamtoperationszeit, die für den Patienten sehr nachteilig, in einzelnen Fällen sogar lebensbedrohlich sein kann. Hiervon sind vor allen Dingen betroffen Schwerverletzte, alte Menschen oder durch sonstige allgemeine Erkrankungen unabhängig von dem notwendigen chirurgischen Eingriff vorbelastete Menschen. Die zusätzliche Operation führt weiterhin zu höherem Blutverlust und ist für den Betroffenen wegen der entstehenden Schmerzen unangenehm. Ausserdem hat jeder operative Eingriff eine gewisse Komplikationsrate, die zu weiteren, im Einzelfall nicht immer vorhersehbaren, Schädigungen führen kann.

2. Die Menge des zur Verfügung stehenden autologen Knochens ist begrenzt.

Die zur Verfügung stehenden Reservoire sind die Beckenkämme beiderseits, Rippen, der Schienbeinkopf und die körperfernen Teile der Speiche. Selbst die vollständige Ausnutzung dieser Quellen, die dann mehrere operative Eingriffe erforderlich macht, liefert nicht in jedem Falle eine ausreichende Menge an Knochenersatzmaterial. Ist der Bedarf grösser, so ist der Operateur gezwungen, entweder nicht ausreichende Mengen Knochenersatzmaterial zu verwenden oder auf weniger gut geeignete Knochen von anderen Menschen oder vom Tier zurückzugreifen.

Bei der homologen Knochentransplantation von einem Menschen zum anderen wird das Transplantat vom Empfänger oftmals nicht gut angenommen. Dafür ist vor allen Dingen die Bildung von Antikörpern gegen fremdes Eiweiss verantwortlich. Geeignetes Spendermaterial ist ausserdem nur schwierig zu bekommen. In der Regel muss man auf von der Leiche entnommenen Knochen zurückgreifen, der in seinem Wert schon wieder stark gemindert ist. Die Übertragung von Infektionen auf den Empfänger ist nicht ausgeschlossen.

Die Verwendung von heterologem Transplantationsmaterial wie z.B. Kieler Knochenspan nach DE-C-961 654 im Laufe vieler Jahre zeigte, dass von einigen Patienten dieses Material nicht gut angenommen wurde. Die vom Chirurgen eingesetzten Knochenstücke wurden häufig nicht zu lebendem Knochengewebe umgebaut, sondern blieben im Gewebe als totes Material liegen.

Der Erfindung liegt daher die Aufgabe zugrunde, ein neues Knochenersatzmaterial zu schaffen, das in beliebiger Menge verfügbar ist, bei der Einpflanzung in den Körper von Mensch oder Tier keine Antikörper erzeugt, gut gewebeverträglich ist und nach Einpflanzung in den Körper zu Knochengewebe umgebaut wird.

Diese Aufgabe wird durch die im Anspruch 1 aufgeführten Merkmale gelöst.

Als Ausgangsmaterial für das erfindungsgemässe Knochenersatzmaterial dienen Knochen und insbesondere Bovines-Knochenmaterial, z.B. vom Kalb. Es können jedoch auch andere Ausgangsmaterialien, wie menschliche Knochen oder ähnliches kollagenhaltiges Material, verwendet werden. Nachstehend wird das Verfahren zur Herstellung des erfindungsgemässen Knochenersatzmaterials anhand von Kalbsknochen als Ausgangsmaterial beispielhaft dargestellt.

Das Verfahren stellt eine Kombination von verschiedenen Stufen dar, die in der angegebenen Reihenfolge durchgeführt werden müssen, um das erfindungsgemässe Knochenersatzmaterial mit verbesserter biologischer Stabilität zu erhalten.

In einer ersten Stufe werden die Knochen zur Entfernung des Blutfarbstoffes und anderer wasserlöslicher Eiweisse über einen gewissen Zeitraum gewässert. Vorzugsweise beträgt dieser Zeitraum 2 - 3 Tage. Bei diesem Wässern wird die Waschflüssigkeit häufig erneuert. Das Wässern kann auch als fliessendes Wässern durchgeführt werden. Eine besondere Temperatur ist nicht erforderlich, doch wird zweckmässig bei Umgebungstemperatur gewässert.

Eine zweite Verfahrensstufe dient der Entfernung weiterer, nicht wasserlöslicher Eiweissanteile. Es ist wichtig, dass fremde Eiweisse bzw. Proteine ausserhalb des Kollagens wie z.B. Knochenzellen entfernt werden, um Antireaktionen durch Antikörperbildung auch enzymatischer Art zu vermeiden. Die Entfernung der Eiweissanteile in dieser Stufe erfolgt durch Einlegen des Knochenmaterials in ein Eiweissentfernungsmittel, das aus einer $H_2O_2$-Lösung besteht. Diese $H_2O_2$-Lösung sollte eine Konzentration von über 10% aufweisen. Die Zeitdauer der Behandlung beträgt normalerweise 2 - 3 Tage. Auch hier muss die Lösung mehrfach erneuert werden, um eine vollständige Entfernung der Eiweissanteile zu gewährleisten.

Die dritte Verfahrensstufe besteht wieder in einem Spülschritt, bei dem das in der zweiten Stufe erhaltene Material mit kaltem Wasser zur Auswaschung und Abführung der gelösten Weichteile gewässert wird. Hier ist das fliessende Wässern von Vorteil.

In der vierten Verfahrensstufe wird das so erhaltene Material entfettet. Das Entfetten erfolgt mit Hilfe von organischen Lösungsmitteln. Geeignete Lösungsmittel sind z.B. Aceton oder Äther oder insbesondere eine Kombination beider Lösungsmittel. Die Entfettung kann in einer beliebigen dafür geeigneten Apparatur vorgenommen werden. Besonders geeignet ist eine Soxhlet-Apparatur für diese Entfettung.

In der fünften Stufe des Verfahrens wird das in der vierten Stufe erhaltene Material mit Hilfe von Komplexbildnern oder Ionenaustauschern behandelt. Diese Stufe dient dem Zweck, den Mineralanteil des Knochens durch Komplexbildung herauszulösen. Geeignete Komplexbildner sind z.B. die Salze von Äthylendiamintetraessigsäure. Eine geeignete Lösung kann nach einem in Romeis «Lehrbuch der mikroskopischen Technik», Seite 401, angegebenen Verfahren hergestellt werden. Dabei werden 250 g Dinatriumäthylendiamintetraessigsäure mit 200 ml destilliertem Wasser versetzt und erwärmt. Unter dauerndem Rühren werden 50 ml einer 40%igen Natronlauge zugesetzt und das Ganze mit destilliertem Wasser auf 800 ml aufgefüllt. Durch tropfenweisen Zusatz von 40%iger Natronlauge wird auf pH 7,4 eingestellt und mit destilliertem Wasser auf 1000 ml Gesamtvolumen aufgefüllt.

Das Knochenmaterial wird in eine solche Lösung eingelegt und verbleibt darin eine geraume Zeit, z.B. bis zu mehreren Wochen, bis ein kautschukartiges elastisches Produkt entstanden ist.

Das so erhaltene kautschukartige elastische Produkt wird in einer sechsten Verfahrensstufe erneut gewaschen, um die vorhandenen Salzreste zu entfernen. Das Waschen geschieht durch fliessendes Wässern für einen ausreichenden Zeitraum, wie z.B. 2 - 3 Tage.

Das dabei erhaltene Material kann dann in einer siebten Verfahrensstufe gegebenenfalls einer Gefriertrocknung in üblicher Weise unterworfen werden.

Das erhaltene Material wird dann in einer achten Verfahrensstufe der üblichen Sterilisation, insbesondere der Strahlensterilisation, unterworfen. Die Strahlensterilisation kann mit üblichen Dosen z.B. mit einer Dosis von 2,5 Megarad erfolgen.

Bei dem oben geschilderten Kombinationsverfahren sind die Stufen 1 bis 4 und die Stufe 5 jeweils an sich bekannt. Allerdings wird gemäss der DE-C-961 654 nach der Arbeitsstufe 3 eine Trocknungsstufe eingeschoben, die bei höchstens 40°C über einen Zeitraum von 12 - 24 Stunden durchgeführt wird. Beim vorliegenden Verfahren darf diese Trocknungsstufe nicht angewandt werden, da man sonst nicht zu dem erfinderischen Ergebnis gelangt. Es ist also wesentlich, dass nach der Verfahrensstufe 3, d.h. dem Wässern, ohne eine Trocknungsstufe die an sich bekannte Arbeitsstufe 4, d.h. das Entfetten mit organischen Lösungsmitteln, durchgeführt wird.

Die Verfahrensstufe 5 ist als solche z.B. aus dem genannten «Lehrbuch der mikroskopischen Technik» von Romais bekannt. Nicht bekannt ist dagegen die Kombination der Verfahrensstufen 1 - 4 der vorliegenden Anmeldung mit der Verfahrensstufe 5 der Anmeldung.

Bei den Verfahrensstufen 6 - 8 handelt es sich um übliche, an sich bekannte Verfahrensstufen. Jedoch sind auch diese Stufen in Kombination mit den Verfahrensstufen 1 - 5 der vorliegenden Anmeldung nicht bekannt.

Durch die erfinderische Kombination der für sich genommen an sich bekannten Einzelverfahrensstufen ist es möglich geworden, ein Knochenersatzmaterial aus gereinigtem Kollagen und insbesondere gereinigtem Kalbskollagen zu erhalten, das durch sorgfältige Vermeidung aller schädigenden Faktoren, wie z.B. Säure beim Entkalkungsprozess, in seiner molekularen Struktur weitgehend erhalten ist.

Das so erhaltene Grundmaterial zeigt bei der Implantation in den Körper als Knochenersatzmaterial erstaunlich gute Eigenschaften. Beispielsweise ist die Resorptionszeit wesentlich höher als bei anderen bisher bekannten Materialien.

Darüber hinaus haben feingewebliche Untersuchungen, die nach Einpflanzung in Versuchstiere zu verschiedenen Zeitpunkten entnommen wurden, eine hervorragende Verträglichkeit ohne Abstossungsreaktionen des Körpers gezeigt. Schon nach 14 Tagen konnte eine beginnende Umwandlung in Knochengeweben nachgewiesen werden. Feingewebliche Untersuchungen zeigten Einlagerungen von Mineralsubstanz, Bindegewebszellen, einsprossende Blutgefässe und knochenbildende Zellen.

Das auf obige Weise erhaltene Grundmaterial kann in seinen hervorragenden Eigenschaften als Knochenersatzmaterial noch wesentlich verbessert werden, wenn man dieses Material mit einer geringen Menge des Hormons Calcitonin, auch Thyreo-Calcitonin genannt, vermischt. Beispielsweise eignen sich dazu im Handel erhältliche Calcitonin-Lösungen. Die wesentlich verbesserten Ergebnisse lassen den Schluss zu, dass es sich hier um einen synergistischen Effekt zwischen dem Knochenersatzgrundmaterial und dem Hormon Calcitonin handelt. Die Knochenneubildungszeit liess sich mit einem derart mit Calcitonin versetzten Knochenersatzmaterial weiter erheblich verkürzen.

Die Erfindung wird nachstehend anhand eines Ausführungsbeispiels erläutert:

1 kg Kalbsknochen (Spongiosastücke) wird sorgfältig gewaschen und so von groben Blutresten befreit. Diese Knochenmenge wird dann in einem geeigneten Gefäss mit Zu- und Abflussmöglichkeit gelagert und für 72 Stunden durch fliessendes Leitungswasser einer Dauerspülung ausgesetzt. Dann wird es in eine $H_2O_2$-Lösung (15%) für 72 Stunden eingelegt. Die Lösung wird 3- bis 4mal gewechselt. Die Behandlung mit fliessendem Leitungswasser wird dann für 24 Stunden wiederholt. Anschliessend wird die Entfettung in einer Soxhlet-Apparatur mit 60% Aceton und 40% Diäthyläther über ca. 12 Stunden vorgenommen.

1250 g Dinatriumäthylendiamintetraessigsäure werden mit 1000 ml destilliertem $H_2O$ versetzt und erwärmt. Unter dauerndem Rühren werden 200 ml NaOH (40%) zugesetzt und das Ganze mit destilliertem Wasser auf 4000 ml aufgefüllt. Durch tropfenweisen Zusatz von 40%iger NaOH wird auf pH 7,4 eingestellt und mit destilliertem Wasser auf 5000 ml aufgefüllt.

In die so erhaltene Lösung werden die wie vorstehend beschrieben behandelten Knochen eingelegt, bis mehrere entnommene Proben sich als mineralfrei erweisen. Bei Zimmertemperatur sind dazu je nach Grösse der Einzelstücke etwa 3 - 4 Wochen erforderlich.

Dann wird zur Entfernung des Restsalzes das erhaltene Material in fliessendem Wasser gewässert. Anschliessend wird gefriergetrocknet. Danach wird nach geeigneter Folienverpackung durch Bestrahlung mit 2,5Mrad sterilisiert.

Im folgenden werden Versuche und ihre Ergebnisse beschrieben, bei denen das Knochenersatzmaterial gemäss der Erfindung implantiert worden ist.

Das erfindungsgemässe Knochenersatzmaterial wurde bei der Ratte und beim Kaninchen auf seine Eigenschaften geprüft.

Die Kollagenmatrix wurde unter die Haut und in die Rückenmuskulatur der Tiere eingepflanzt. Bei verschiedenen Gruppen wurden die Implantate nach 1, 2, 4, 8 und 12 Wochen entfernt und histologisch untersucht.

Nach 1 Woche war bereits das Einwachsen von feinsten Blutgefässen und Zellen erkennbar.

Nach 14 Tagen konnten Chondroblasten, Fibroblasten und Osteoblasten nachgewiesen werden (knorpel-, bindegewebe- und knochenbildende Zellen).

Im weiteren Verlauf erfolgte fortschreitender Auf- und Umbau bis zur Ausbildung regulärer Knochenstrukturen.

## Patentansprüche

1. Verfahren zur Herstellung von Knochenersatzmaterial mit verbesserter biologischer Stabilität unter Verwendung von tierischem oder menschlichem Knochenmaterial als Ausgangssubstanz, bei dem man

a) den Blutfarbstoff und andere wasserlösliche Eiweisse aus dem Knochenmaterial durch Behandeln mit Wasser entfernt, anschliessend

b) nicht wasserlösliches Eiweiss und Proteine durch Behandeln mit $H_2O_2$-Lösung entfernt, dann

c) in kaltem Wasser spült, danach

d) mit organischem Lösungsmittel entfettet und

e) das erhaltene Material in üblicher Weise sterilisiert, dadurch gekennzeichnet, dass man

f) das Entfetten unmittelbar nach dem auf die Eiweissentfernung folgenden Spülen mit kaltem Wasser durchführt,

g) anschliessend die Mineralanteile durch Komplexbildner oder Ionenaustauscher herauslöst, und

h) zur Entfernung des Restsalzes das erhaltene Material in fliessendem Wasser wässert.

2. Verfahren nach Anspruch 1, dadurch gekennzeichnet, dass als Ausgangsmaterial Bovines-Knochenmaterial, z.B. Knochen vom Kalb, verwendet wird.

3. Verfahren nach Anspruch 1 oder 2, dadurch gekennzeichnet, dass dem Knochenersatzmaterial zusätzlich das Hormon Calcitonin zugegeben wird.

4. Verfahren nach einem der Ansprüche 1 bis 3, dadurch gekennzeichnet, dass man das erhaltene Material vor dem Sterilisieren in üblicher Weise gefriertrocknet.

5. Verfahren nach einem der Ansprüche 1 bis 4, dadurch gekennzeichnet, dass die Sterilisation durch Bestrahlen erfolgt.

6. Knochenersatzmaterial mit verbesserter biologischer Stabilität, hergestellt gemäss einem der vorangehenden Verfahrensansprüche 1 bis 5, dadurch gekennzeichnet, dass das Knochenersatzmaterial aus natürlichem, entmineralisiertem Kollagen mit weitgehend erhaltener molekularer Struktur besteht.

## Claims

1. A process for producing a bone substitute material having an improved biological stability by using animal or human bone material as the starting substance, which includes

a) removing the blood pigment and other water-soluble proteins from the bone material by treatment with water; subsequently

b) removing water-insoluble proteins by treatment with $H_2O_2$ solution; then

c) rinsing in cold water; then

d) degreasing with an organic solvent; and

e) sterilizing the material obtained by a conventional method,

characterized in that

f) the step of degreasing is carried out immediately after the step of rinsing in cold water which follows upon the removal of proteins,

g) the mineral content is subsequently eliminated by dissolution with a complexing agent or an ion exchanger, and

h) the material obtained is rinsed in running water in order to remove the residual salts therefrom.

2. A process according to claim 1, characterized in that a bovine bone material, e.g. calf bones, is used as the starting material.

3. A process according to claim 1 or 2, characterized in that the hormone calcitonin is further added to the bone substitute material.

4. A process according to any of claims 1 to 3, characterized in that the material obtained is freeze-dried by a conventional method prior to sterilization.

5. A process according to any of claims 1 to 4, characterized in that the sterilization is accomplished by irradiation.

6. Bone substitute material having an improved biological stability, and having been produced according to any of the preceding process claims 1 to 5, characterized in that the bone substitute material consists of natural demineralized collagen, the molecular structure of which has been preserved to a large extent.


**Revendications**

1. Procédé pour l'obtention d'un matériau de remplacement des os ayant une meilleure stabilité biologique, comportant l'utilisation d'un matériau osseux, animal ou humain, comme substance de départ, et dans lequel:

a) on sépare l'hémoglobine et d'autres protides solubles dans l'eau, du matériau osseux, par un traitement à l'eau, puis

b) on sépare l'albumine et les protéines insolubles dans l'eau par un traitement avec une solution de $H_2O_2$,

c) on rince ensuite à l'eau froide,

d) on dégraisse ensuite avec un solvant organique, et

e) on stérilise de façon classique le matériau obtenu,

caractérisé en ce que

f) on effectue le dégraissage directement après le rinçage à l'eau froide suivant la séparation de l'albumine,

g) puis on sépare les constituants minéraux par formation de complexe ou par un échangeur d'ions, et

h) on lave le matériau obtenu à l'eau courante pour éliminer le sel résiduel.

2. Procédé selon la revendication 1, caractérisé en ce que comme matériau de départ on utilise un matériau à base d'os de bovins, par exemple des os de veau.

3. Procédé selon l'une des revendications 1 ou 2, caractérisé en ce qu'au matériau de remplacement des os, on ajoute en outre l'hormone calcitonine.

4. Procédé selon l'une quelconque des revendications 1 à 3, caractérisé en ce qu'on lyophilise le matériau obtenu, de façon usuelle, avant la stérilisation.

5. Procédé selon l'une quelconque des revendications 1 à 4, caractérisé en ce que la stérilisation est effectuée par irradiation.

6. Matériau de remplacement des os ayant une meilleure stabilité biologique, préparé selon l'une quelconque des revendications de procédé précédentes 1 à 5, caractérisé en ce que le matériau de remplacement des os est constitué de collagène naturel, déminéralisé, avec une structure notablement moléculaire.